# EUROPEAN PATENT APPLICATION

(11) **EP 4 682 244 A1**
(43) Date of publication of application: **21.01.2026**
(21) Application number: 24852931.5
(22) Date of filing: 19.06.2024
(51) Int. Cl.: C12N 1/20, A61K 35/741, A61K 35/745, A61P 3/14, A61P 19/10, A61P 1/00

(54) **PROBIOTIC AGENT CONTAINING AKK11 STRAIN FOR PROMOTING CALCIUM ABSORPTION AND USE THEREOF**

(30) Priority: 20.05.2024 CN 202410620734
(71) Applicant: Wecare Probiotics Co., Ltd., Suzhou, Jiangsu 215200 (CN)
(72) Inventor: FANG, Shuguang, Suzhou, Jiangsu 215200 (CN); DONG, Yao, Suzhou, Jiangsu 215200 (CN); GU, Jiayue, Suzhou, Jiangsu 215200 (CN); WU, Zhiyi, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Buzzi, Notaro & Antonielli d'Oulx S.p.A.
(86) International application number: PCT/CN2024/100150
(87) International publication number: WO 2025/241246

(57) **Abstract**

Provided are a probiotic preparation containing an Akk11 strain for promoting calcium absorption and a use thereof, and the probiotic preparation for promoting calcium absorption includes an *Akkermansia muciniphila* Akk11 strain and a *Bifidobacterium breve* BBr16 strain. The present application developed a novel compounding method for probiotics, and found potential interactions between the Akk11 strain and BBr16 strain that these two strains can cooperate with each other to synergistically enhance the effect of promoting calcium absorption, specifically manifested in: the overall metabolism ability of calcium can be improved through the improvement of intestinal health and the enhancement of the absorption capacity of the intestinal wall; the serum calcium level and phosphorus content can be promoted and restore a normal electrolyte level; and the loss of calcium in bones can be effectively reduced, thereby stabilizing the structure and function of the bones.

## Description

### TECHNICAL FIELD

The present application belongs to the technical field of probiotic preparations, and relates to a probiotic preparation containing an Akk11 strain for promoting calcium absorption and a use thereof.

### BACKGROUND

Calcium is one of the most abundant minerals in the human body and is essential for maintaining physiological processes such as bone health, nerve conduction, muscle function, and blood clotting. Insufficient calcium absorption is associated with various health problems, including osteoporosis, increased risks of fracture, and growth retardation. Calcium absorption occurs primarily in the small intestine and is influenced by a number of factors, including the form of calcium in the diet, intestinal pH, other nutrients present in the gut, and the state of the intestinal microflora.

Gut microbes, especially probiotics, have a potential role in promoting calcium absorption. Probiotics can affect calcium absorption through a variety of mechanisms, including altering intestinal pH, promoting the expression of calbindin, reducing inflammation by enhancing intestinal barrier integrity and contributing to improved nutrient absorption, and potentially indirectly affecting calcium absorption and metabolism by regulating the composition of the intestinal microflora.

The use of probiotics in promoting calcium absorption is still in the preliminary stage with few strategies. Therefore, the development of more probiotic preparations that can effectively promote calcium absorption can not only provide new strategies to solve the problem of insufficient calcium absorption, but also help to deeply understand the interactions between gut microbes and host nutrient metabolism, and provide scientific basis and technical support for the prevention and promotion of insufficient calcium absorption.

### SUMMARY

The present application provides a probiotic preparation containing an Akk11 strain for promoting calcium absorption and a use thereof, and specifically relates to a probiotic preparation containing an Akk11 strain for promoting calcium absorption and a use thereof in the preparation of a product for preventing or ameliorating insufficient calcium absorption or in the preparation of a product for preventing, ameliorating, or treating a disease caused by insufficient calcium absorption.

In a first aspect, the present application provides a probiotic preparation containing an Akk11 strain for promoting calcium absorption. The probiotic preparation for promoting calcium absorption includes an *Akkermansia muciniphila* Akk11 strain and a *Bifidobacterium breve* BBr16 strain, wherein the *Akkermansia muciniphila* Akk11 strain is deposited in China Center for Type Culture Collection on Jan. 15, 2024, with the deposit number of CCTCC NO. M2024119, and the address of Wuhan University, Wuhan, China; and the *Bifidobacterium breve* BBr16 is deposited in China General Microbiological Culture Collection Center on Mar. 7, 2022, with the deposit number of CGMCC No. 24471, and the address of No. 3, Courtyard 1, West Beichen Road, Chaoyang District, Beijing.

The present application developed a novel compounding method for probiotics, which compounds the *Akkermansia muciniphila* Akk11 strain and the *Bifidobacterium breve* BBr16 strain, and potential interactions have been found that these two strains can cooperate with each other to synergistically enhance the effect of promoting calcium absorption, specifically manifested in: (1) the overall metabolism ability of calcium can be improved through the improvement of intestinal health and the enhancement of the absorption capacity of the intestinal wall; (2) the serum calcium level and phosphorus content can be promoted and restore a normal electrolyte level; and (3) the loss of calcium in bones can be effectively reduced, thereby stabilizing the structure and function of the bones.

In the condition of a same amount of bacteria used, compared with a single Akk11 strain or a single BBr16 strain, the compounding of the two bacteria in the above effect can be significantly improved. Therefore, the probiotic preparation provides a new strategy for preventing, relieving or treating a related disease caused by insufficient calcium absorption. Since *Akkermansia muciniphila* and *Bifidobacterium breve* are both probiotics, they have high safety and less dependence when used in the preparation of related efficacy products.

Preferably, a viable bacteria count ratio of the Akk11 strain to the BBr16 strain is 1:10-10:1, for example, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1 or 10:1. Other specific point values within the above numeric range are also applicable, which are not recited here for brevity.

Preferably, a total number of viable bacteria in the probiotic preparation is not less than 1 × 10⁸ CFU/mL or 1 × 10⁸ CFU/g, for example, 1 × 10⁸ CFU/mL (CFU/g), 1 × 10⁹ CFU/mL (CFU/g), 5 × 10⁹ CFU/mL (CFU/g), 1 × 10¹⁰ CFU/mL (CFU/g), 5 × 10¹⁰ CFU/mL (CFU/g), 1 × 10¹¹ CFU/mL (CFU/g), 1 × 10¹² CFU/mL (CFU/g) or 1 × 10¹³ CFU/mL (CFU/g). Other specific point values within the above numeric range are also applicable, which are not recited here for brevity.

Preferably, a dosage form of the probiotic preparation includes a solution agent, a lyophilized powder agent, a capsule agent, a tablet agent or a granule agent. The dosage form of the probiotic preparation involved in the present application is not limited and includes the most commonly used solution agent and lyophilized powder agent, or further prepared capsule agent, tablet agent or granule agent. The lyophilized powder agent can be prepared by the following method:
the Akk11 strain and BBr16 strain are inoculated in a culture medium for cultivation respectively, to obtain a culture solution; the culture solution is centrifuged to obtain bacterial cells; the bacterial cells are resuspended with a lyophilizing protectant to obtain a resuspended solution; and the resuspended solution is lyophilized to obtain the lyophilized powder, then these two lyophilized powders are compounded in proportion; or
the Akk11 strain and BBr16 strain are inoculated in a culture medium for cultivation respectively, to obtain a culture solution; the culture solution is centrifuged to obtain bacterial cells; these two bacterial cells are resuspended with a lyophilizing protectant after mixed in proportion, to obtain a resuspended solution; and the resuspended solution is lyophilized to obtain the lyophilized powder agent.

Preferably, the lyophilizing is performed using a vacuum freezing method.

Preferably, the probiotic preparation further includes a lyophilizing protectant and/or an auxiliary additive.

Preferably, the lyophilizing protectant includes any one or a combination of at least two of skimmed milk, sucrose, lactose, trehalose, dextran, gelatin, dextrine, Arabic gum, sodium alginate, polyvinyl pyrrolidone, sorbitol or xylitol.

Preferably, the auxiliary additive includes any one or a combination of at least two of inulin, oligofructose, oligoxylose, oligogalactose, iso-malto-oligosaccharide, soybean oligosaccharide, *Spirulina, Arthrospira,* Coriolus versicolor polysaccharide, stachyose, polyglucose, α-lactalbumin, or lactoferrin.

In a second aspect, the present application provides a use of the probiotic preparation for promoting calcium absorption as described in the first aspect in the preparation of a product for preventing or ameliorating insufficient calcium absorption.

Preferably, the product further includes an auxiliary material.

The auxiliary material includes any one or a combination of at least two of an excipient, a filler, a binder, a wetting agent, a disintegrant, an emulsifier, a cosolvent, a solubilizer, an osmotic pressure regulator, a colorant, a pH regulator, an antioxidant, a bacteriostat, or a buffering agent.

In a third aspect, the present application provides a use of the probiotic preparation for promoting calcium absorption as described in the first aspect in the preparation of a product for preventing, ameliorating, or treating a disease caused by insufficient calcium absorption.

Preferably, the product further includes an auxiliary material.

The auxiliary material includes any one or a combination of at least two of an excipient, a filler, a binder, a wetting agent, a disintegrant, an emulsifier, a cosolvent, a solubilizer, an osmotic pressure regulator, a colorant, a pH regulator, an antioxidant, a bacteriostat, or a buffering agent.

Compared to the prior art, the present application has the beneficial effects as follows.

The present application develops a novel compounding method for probiotics, which compounds the *Akkermansia muciniphila* Akk11 strain and the *Bifidobacterium breve* BBr16 strain, and potential interactions have been found that these two strains can cooperate with each other to synergistically enhance the effect of promoting calcium absorption, specifically manifested in: (1) the overall metabolism ability of calcium can be improved through the improvement of intestinal health and the enhancement of the absorption capacity of the intestinal wall; (2) the serum calcium level and phosphorus content can be promoted and restore a normal electrolyte level; and (3) the loss of calcium in bones can be effectively reduced, thereby stabilizing the structure and function of the bones.

### BRIEF DESCRIPTION OF DRAWINGS

FIG. 1 is a graph showing statistical results of the weight of rats in each group.
FIG. 2 is a graph showing statistical results of the apparent calcium absorption rate in each group of rats.
FIG. 3 is a graph showing statistical results of the calcium accumulation rate in each group of rats.
FIG. 4 is a graph showing statistical results of the serum calcium level in each group of rats.
FIG. 5 is a graph showing statistical results of the serum phosphorus level in each group of rats.
FIG. 6 is a graph showing statistical results of the content of serum alkaline phosphatase in each group of rats.
FIG. 7 is a graph showing statistical results of the bone calcium level in each group of rats.

### DETAILED DESCRIPTION

The technical solutions of the present application are further described below via specific embodiments. It should be clear to those skilled in the art that the examples are merely used for a better understanding of the present application and should not be regarded as a specific limitation to the present application.

The medium formulations involved in the following embodiments are as follows:
MRS medium: peptone 10 g/L, beef paste 10 g/L, glucose 20 g/L, sodium acetate 2 g/L, yeast powder 5 g/L, diammonium hydrogen citrate 2 g/L, K₂PO₄ · 3H₂O 2.6 g/L, MgSO₄ · 7H₂O 0.1 g/L, MnSO₄ 0.05 g/L, polysorbate 80 (Tween 80) 1 mL/L, cysteine hydrochloride 0.5 g/L.

The binomial nomenclature of the Akkll strain involved in the following embodiments is *Akkermansia muciniphila* Akk11, which is deposited in China Center for Type Culture Collection on Jan. 15, 2024, with the deposit number of CCTCC NO: M2024119, and the address of Wuhan University, Wuhan, China.

The binomial nomenclature of the BBr16 strain involved in the following embodiments is *Bifidobacterium breve,* which is deposited in China General Microbiological Culture Collection Center on Mar. 7, 2022, with the deposit number of CGMCC No. 24471, and the address of No. 3, Courtyard 1, West Beichen Road, Chaoyang District, Beijing.

A preparation method of a bacterial suspension involved in the following: the required strain is inoculated in a liquid culture medium, cultured at 37°C for 24 h for activation, the activation is performed twice continuously to obtain an activation liquid; the activation liquid is inoculated in a liquid culture medium at an inoculate dosage of 5% (v/v), cultured at 37°C for 24 h to obtain a bacterial liquid; the bacterial liquid is centrifuged at 4 °C for 10 min with a rotational speed of 5000 rpm, filtered to obtain bacterial cells; and the bacterial cells are resuspended by a PBS solution to obtain the bacterial suspension.

The data of the test results are statistically analyzed using ggplot2 in R. Compared with the control group, ### stands for p < 0.001, ## stands for p < 0.01, # stands for p < 0.05; compared with the model group, *** stands for p < 0.001, ** stands for p < 0.01, * stands for p < 0.05, and NS. stands for no significant difference.

### Example

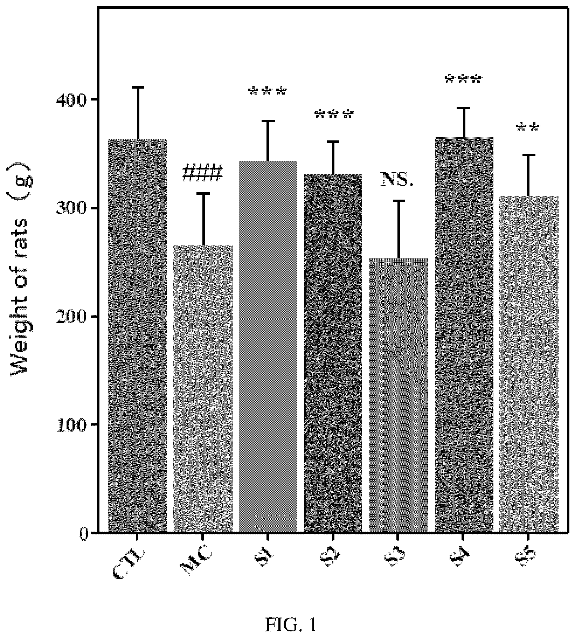

This example explores the symptom improvement ability of a strain on a rat model of calcium deficiency.
(1) Test animals: four-week-old SD rats (56 rats, weighing 75-85 g, purchased from the Shanghai Lab. Animal Center), half male and half female, were fed in a controlled environment, with the room temperature maintained at 20-24°C, humidity at 50-60%, and following a 12 h light/dark cycle. They can eat and drink freely. All experimental procedures involving the rats were in accordance with the ethical guidelines for animal care and use set forth by the Shanghai Laboratory Animal Care and Animal Experimentation Center.
(2) Feed formulation: corn starch 39.8%, casein 19.2%, maltodextrin 13.5%, sucrose 10%, cellulose 5%, soybean oil 7.5%, mineral mixture 3.5% (calcium carbonate was used as the source of calcium, wherein the calcium content of the normal feeds was set at 5000 mg/kg, and the calcium content of the calcium-deficient feeds was lowered to 1000 mg/kg), and mixed vitamins 1%, DL-methionine 0.25%, and choline bitartrate 0.25%.
(3) Animal grouping: after 1 week of adaptive feeding, rats were randomly assigned to 7 groups: control group (CTL), calcium-deficiency model group (MC group), Akk11 strain group (Akk11 group, recorded as S1 group), BBr16 Strain Group (BBr16 group, recorded as S2 group), marketed *Akkermansia muciniphila* group (BNCC341917 group, recorded as S3 group), complexed strains group 1 (Akk11 + BBr16 group, the ratio of viable bacteria count was 2:1, recorded as S4 group), complexed strains group 2 (BNCC341917 + BBr16 group, the ratio of viable bacteria count was 2:1, recorded as S5 group).
(4) Animal modeling and intervention method:
   the CTL group was fed with normal feed + normal purified water, the MC group was fed with calcium-deficient feed + normal purified water, and the S1-S5 groups were fed with calcium-deficient feed + probiotic solution (dosage of 10⁸ CFU/day/individual); all groups were continuously fed for 8 weeks.
(5) Index analysis
   (5.1) Effect on weight of rats
The weights of the rats in each group were measured after the test, and the results are shown in FIG. 1, from which it can be seen that the weights of the rats in MC group decreased significantly compared with those in CTL group; however, the weights of the calcium-deficient rats were reversed and tended to the normal level after the probiotic intervention compared with those in MC group, indicating that the probiotic preparation involved in the present application can help to alleviate the weight loss induced by calcium deficiency, especially in S4 group where the performance was most significant.
(5.2) Research on calcium metabolic balance

Three days before the end of the intervention, the rats in each group were placed in separate metabolic cages and their food intake was recorded daily, while fecal and urine samples were collected. After digestion treatment, the calcium content of the samples was measured using flame atomic absorption spectrometry method. By calculating the amount of calcium ingested (Vi), the amount of calcium in feces (Vf), and the amount of calcium in urine (Vu), the apparent calcium absorption rate and the calcium accumulation rate can be obtained. Based on the amount of calcium ingested, calcium in feces, and calcium in urine, the apparent calcium absorption value (Vab) and calcium accumulation value (Vac) can be obtained.

Apparent calcium absorption rate (%) = (Vab / Vi) × 100 = [(Vi - Vf) / Vi] × 100.

Calcium accumulation rate (%) = (Vac / Vi) × 100 = [(Vi - Vf - Vu) / Vi] × 100.

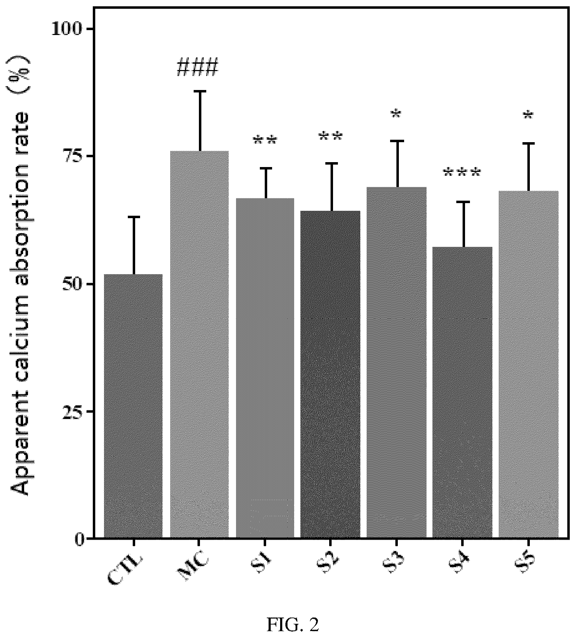

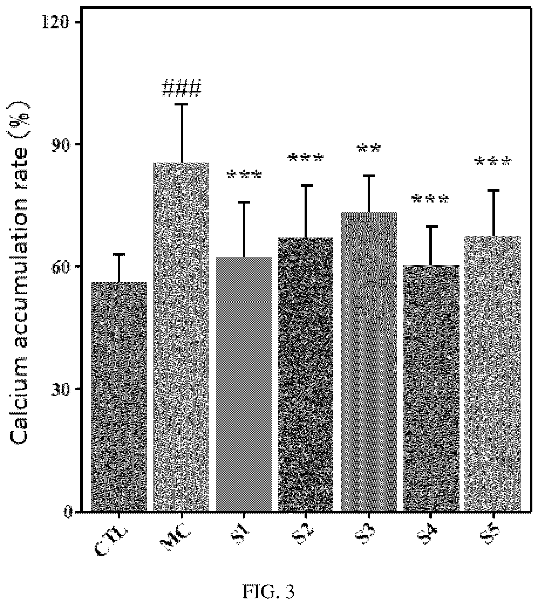

The result is shown in FIG. 2 and FIG. 3, from which it can be seen that the rats in MC group exhibited significant changes in calcium metabolism compared to that of the CTL group, with a significant increase in the apparent calcium absorption rate and calcium accumulation rate, indicating that under the stress of calcium deficiency, the regulatory mechanism in the rats were activated to optimize and improve the absorption of calcium and retention of calcium in the body, such enhanced absorption efficiency and improved calcium accumulation strategy may be a biological adaptive response to maintain physiological demand for calcium and support critical functions. After the probiotic intervention, a gradual return of apparent calcium absorption rate and calcium accumulation rate to near-normal levels was observed, indicating that the probiotic preparation involved in the present application can improve the overall metabolism ability of calcium through the improvement of intestinal health and the enhancement of the absorption capacity of the intestinal wall, especially in S4 group.

### (5.3) Measurement of blood biochemical indicators

After the intervention, 200 µL of blood was collected from each rat at 10-11 am. After centrifugation at 1200 × g for 10 min, serum was extracted and stored at -80°C. Serum calcium level, serum phosphorus level and serum alkaline phosphatase content were quantitatively measured using an enzyme-linked immunosorbent assay kit (Wuhan Chundu Biotech. Co., Ltd.) following the manufacturer's instructions.

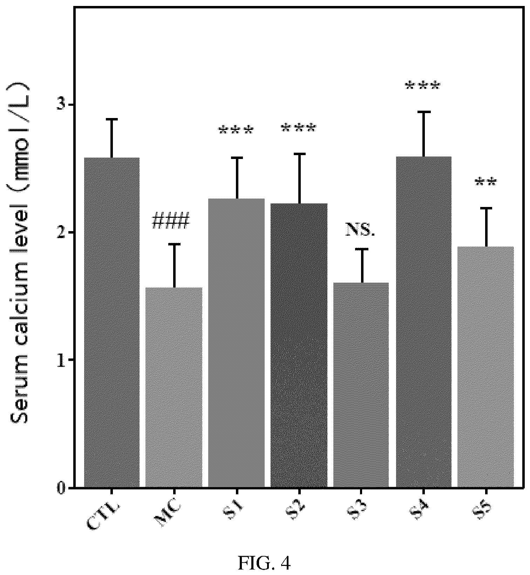

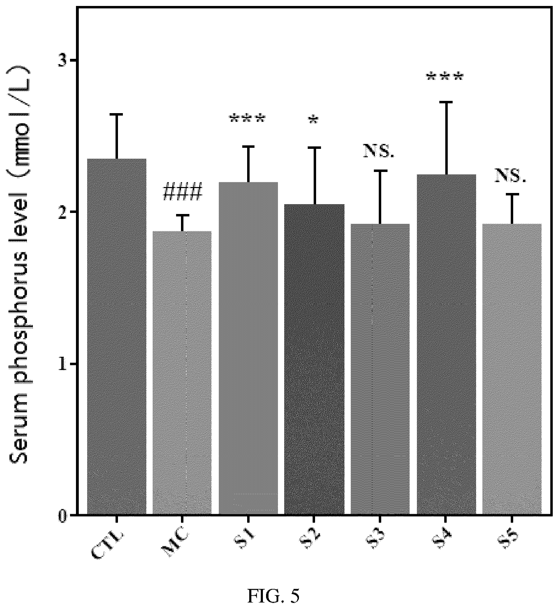

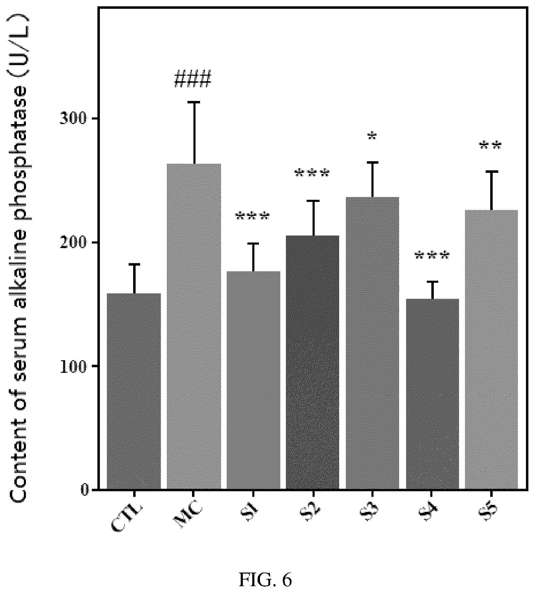

The results are shown in FIGS. 4-6, from which it can be seen that serum calcium level and serum phosphorus level were significantly decreased in the rats of the MC group compared to those of the CTL group, indicating that the calcium deficiency state adversely affects mineral metabolism, leading to a decrease of the concentration of these key electrolytes in blood. In addition, the content of serum alkaline phosphatase was significantly increased in the rats of the MC group, which is usually associated with the enhanced bone metabolic activity, reflecting that bones were attempted to release more minerals to compensate for deficiency in blood. After the probiotic intervention, the serum calcium level and serum phosphorus level of calcium-deficient rats increased significantly, indicating that the intervention of the probiotic preparation of the present application helps to restore the normal electrolyte level and can achieve a better utilization of minerals by improving the intestinal absorption function. In addition, the content of the serum alkaline phosphatase of calcium-deficient rats was significantly reduced, indicating a gradual stabilized bone metabolism process with no need of excessive mobilization of the bone minerals to maintain mineral balance in blood, especially in S4 group where the performance was most significant.

### (5.4) Measurement of rat femur indicators

After the intervention, the muscle and connective tissue of the right femur of the rat were completely stripped, and the treated femur was dried at 95°C in order to undergo a dry ashing treatment, which was performed as follows: ashing was performed at 600°C for 8 h, and the ashed sample was dissolved and treated by using 6 mol/L hydrochloric acid, and the content of calcium in bone was determined by atomic absorption spectroscopy.

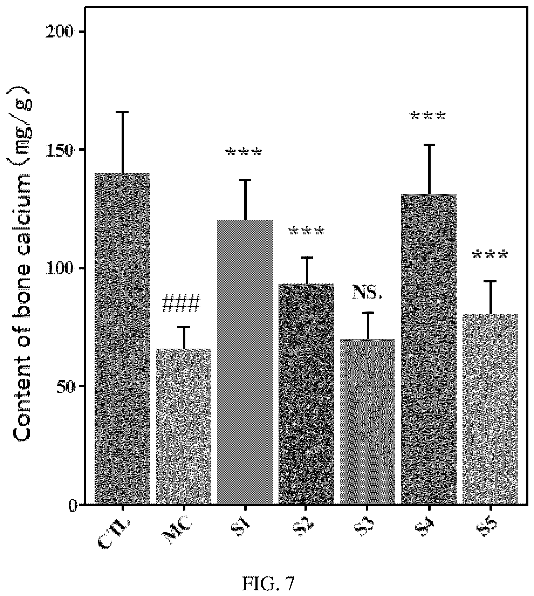

The result is shown in FIG. 7, from which it can be seen that the bone calcium level of rats in MC group was significantly decreased compared with to that of the CTL group, which was a typical manifestation of the calcium deficiency condition directly affecting the bone mineral density, and such a decrease may be due to the loss of calcium in bone caused by insufficient calcium intake, which in turn affects the structure and function of the bone. However, the content of bone calcium in calcium-deficient rats was significantly increased after the intervention of probiotic of the present application, indicating that a positive effect of the addition of probiotic on improving bone health, especially in S4 group, and may enhance the bioavailability of calcium through the improvement of intestinal health condition and enhancement of the absorption function of the intestinal wall. This allows more calcium to be absorbed into the bloodstream for body use, including the use for the mineralization process of the bones, helping to slow down the loss rate of bones and enhancing the structural strength and density of the bones by improving the overall calcium balance.

The applicant declares that the present application illustrates the detailed technical solutions of the present application by the above embodiments, but the present application is not limited to the above embodiments, that is, the present application does not necessarily rely on the above embodiments to be implemented. Those skilled in the art should understand that any improvements of the present application, the equivalent substitution of each raw material, the addition of auxiliary ingredients, and the selection of specific methods shall fall within the protection scope and disclosure scope of the present application.

The above describes in detail the preferred embodiments of the present application. However, the present application is not limited to the specific details in the above embodiments, and various simple variations of the technical solutions of the present application can be made within the scope of the technical conception of the present application, all of these simple variations shall fall within the protection scope of the present application.

It is also to be noted that the various specific technical features described in the above specific embodiments may be combined in any suitable manners without contradiction, and in order to avoid unnecessary repetition, the various possible combinations are not described separately in the present application.

## Claims

1. A probiotic preparation containing an Akk11 strain for promoting calcium absorption, which comprises an *Akkermansia muciniphila* Akk11 strain with a deposit number of CCTCC NO: M2024119 and a *Bifidobacterium breve* BBr16 strain with a deposit number of CGMCC No.24471.

2. The probiotic preparation for promoting calcium absorption according to claim 1, wherein a viable bacteria count ratio of the Akk11 strain to the BBr16 strain is 1:10-10:1.

3. The probiotic preparation for promoting calcium absorption according to claim 1, wherein a total number of viable bacteria in the probiotic preparation is not less than 1 × 10⁸ CFU/mL or 1 × 10⁸ CFU/g.

4. The probiotic preparation for promoting calcium absorption according to claim 1, wherein a dosage form of the probiotic preparation comprises a solution agent, a lyophilized powder agent, a capsule agent, a tablet agent or a granule agent.

5. The probiotic preparation for promoting calcium absorption according to claim 1, wherein the probiotic preparation further comprises a lyophilizing protectant and/or an auxiliary additive.

6. The probiotic preparation for promoting calcium absorption according to claim 5, wherein the lyophilizing protectant comprises any one or a combination of at least two of skimmed milk, sucrose, lactose, trehalose, dextran, gelatin, dextrine, Arabic gum, sodium alginate, polyvinyl pyrrolidone, sorbitol or xylitol.

7. The probiotic preparation for promoting calcium absorption according to claim 5, wherein the auxiliary additive comprises any one or a combination of at least two of inulin, oligofructose, oligoxylose, oligogalactose, iso-malto-oligosaccharide, soybean oligosaccharide, *Spirulina, Arthrospira,* Coriolus versicolor polysaccharide, stachyose, polyglucose, α-lactalbumin, or lactoferrin.

8. Use of the probiotic preparation for promoting calcium absorption according to any one of claims 1-7 in the preparation of a product for preventing or ameliorating insufficient calcium absorption.

9. Use of the probiotic preparation for promoting calcium absorption according to any one of claims 1-7 in the preparation of a product for preventing, ameliorating, or treating a disease caused by insufficient calcium absorption.

10. The use according to claim 9, wherein the product further comprises an auxiliary material; the auxiliary material comprises any one or a combination of at least two of an excipient, a filler, a binder, a wetting agent, a disintegrant, an emulsifier, a cosolvent, a solubilizer, an osmotic pressure regulator, a colorant, a pH regulator, an antioxidant, a bacteriostat, or a buffering agent.
